Europäisches Patentamt

European Patent Office

Office européen des brevets

(19)

(11) Numéro de publication: **0 030 897**
**B2**

(12) # NOUVEAU FASCICULE DE BREVET EUROPEEN

(45) Date de publication du nouveau fascicule du brevet:
26.08.87

(51) Int. Cl.⁴: **A 61 N 1/362**

(21) Numéro de dépôt: **80401795.2**

(22) Date de dépôt: **12.12.80**

(54) Stimulateur cardiaque implantable polyvalent.

(30) Priorité: **18.12.79 FR 7930930**

(43) Date de publication de la demande:
**24.06.81 Bulletin 81/25**

(45) Mention de la délivrance du brevet:
**25.04.84 Bulletin 84/17**

(45) Mention de la décision concernant l'opposition:
**26.08.87 Bulletin 87/35**

(84) Etats contractants désignés:
**AT BE CH DE GB IT LI LU NL SE**

(56) Documents cités:
**WO - A - 79/00070**
**FR - A - 1 440 902**
**FR - A - 1 515 162**
**FR - A - 2 133 835**
**FR - A - 2 379 104**
**FR - A - 2 383 674**
**GB - A - 2 026 870**
**US - A - 2 478 746**
**US - A - 3 669 120**

**Telefunken Laborbuch, Band 3, 2. Ausgabe 1966, Seiten 72 bis 90**

(73) Titulaire: **CARDIOFRANCE- COMPAGNIE FRANCAISE D'ELECTROCARDIOLOGIE, 40, Grande Rue, F-95450 Commeny (FR)**

(72) Inventeur: **Buffet, Jacques, 20, avenue Thiers, F-93340 Le Raincy (FR)**

(74) Mandataire: **Derambure, Christian, Cabinet BUGNION ASSOCIES SARL 116, boulevard Haussmann, F-75008 Paris (FR)**

## Description

La présente invention concerne un stimulateur cardiaque implantable polyvalent.

Les stimulateurs cardiaques, implantables sont déjà très largement connus. Ils comprennent généralement un boîtier conducteur de l'électricité, susceptible de constituer une borne; une source d'énergie électrique logée dans le boîtier; des circuits et composants électroniques associés à la source d'énergie électrique, également logés dans le boîtier, propres à délivrer des impulsions de stimulation du cœur d'un patient recevant ledit stimulateur, comportant notamment un amplificateur d'entrée et un amplificateur de sortie; deux sorties, chacune monopolaire, associées aux circuits électroniques et auxquelles peuvent être associées deux électrodes monopolaires de stimulation.

Dans cette forme d'execution, les deux bornes des électrodes de stimulation sont implantées en deux points prédéterminés du cœur du patient. En particulier, ces deux points appartiennent à une même zone du cœur, notamment le ventricule.

Jusqu'à présent, un tel stimulateur cardiaque ainsi implanté est destiné à fonctionner selon le mode dit bipolaire et le mode dit monopolaire double. Dans le mode bipolaire, la borne constituée par le boîtier du stimulateur est neutre et les impulsions de stimulations interviennent entre les deux bornes associées aux deux électrodes. Dans le mode dit monopolaire double, les impulsions interviennent entre la borne constituée par le boîtier, lequel n'est donc pas neutre et chacune des deux bornes des deux électrodes. Le même stimulateur est susceptible de fonctionner selon le mode monopolaire. Dans ce dernier cas, l'impulsion intervient entre la borne constituée par le boîtier et une seulement des deux bornes des deux électrodes. Ainsi, dans ce dernier cas, une seule électrode est active tandis que l'autre électrode est passive.

Jusqu'à ce jour, le mode de fonctionnement du stimulateur est déterminé préalablement à son implantation et l'opérateur organise le stimulateur cardiaque à cet effet. Il en résulte qu'il n'est guère possible une fois le stimulateur cardiaque implanté de passer d'un mode de fonctionnement à un autre mode par simple commande externe. D'autre part, il a été considéré que d'autres modes de fonctionnement pouvaient être utiles notamment les modes dits double monopolaire d'une part et «bifocal ventriculaire» d'autre part. On entend ici par mode double monopolaire le mode dans lequel le stimulateur du type décrit plus haut comporte deux électrodes, une première électrode permettant un fonctionnement selon le mode monopolaire et celui-ci pouvant aussi être assuré, ultérieurement et en cas de besoin par l'autre électrode. Par mode bifocal ventriculaire on entend un mode dans lequel le stimulateur du type décrit plus haut comporte deux électrodes dont l'une est essentiellement utilisée pour la réception de signaux en provenance du cœur du patient alors que l'autre est utilisée pour l'envoi des excitations de stimulation. Dans ce mode bifocal ventriculaire, chaque électrode fonctionne de façon unidirectionnelle alors que dans le mode monopolaire déjà mentionné plus haut, l'électrode active fonctionne de façon bidirectionnelle tant pour la réception que pour l'envoi des signaux de stimulation.

Le brevet français no 2 383 674 décrit un stimulateur pouvant fonctionner suivant plusieurs modes différents. Dans au moins un mode de fonctionnement, on établit des parcours de signaux différents de ceux d'un premier mode de fonctionnement. Des moyens sont prévus pour émettre des impulsions de durée, d'amplitude et/ou de fréquence de récunence prédéterminées. Des moyens permettent de déclencher, lors du premier ou du second mode, des signaux en réponse à l'apparition de ces impulsions et des moyens, lors du second mode, déclenchent ou inhibent des signaux à la fin des impulsions ou pendant leur durée. Les signaux fixent les instants d'apparition des stimuli émis par le stimulateur. Les moyens d'impulsions sont constitués par un circuit multivibrateur qui peut être déclenché par un signal d'entrée provenant du cœur. Le second mode est par exemple tel que le stimulateur est commandé par une oreillette ou tel que le stimulateur fonctionne en stimulateur sentinelle.

Le brevet français no. 1 440 902 décrit un stimulateur dans lequel on utilise un commutateur magnétique pour remplacer une électrode détériorée par une électrode de rechange. Ainsi, par le jeu du champ magnétique, on peut mettre en ou hors circuit l'électrode du stimulateur.

La demande de brevet internationale WO 79/00070 décrit un stimulateur cardiaque qui comporte deux électrodes et un commutateur interne qui sélectionne, soit automatiquement, soit grâce à des moyens de commande externes, l'une des deux électrodes qui fonctionne tant pour l'envoi que pour la réception des signaux. En variante, le stimulateur cardiaque comporte deux commutateurs, associés chacun à une électrode. Dans cette variante, une des électrodes peut être utilisée pour la réception des signaux, tandis que l'autre est utilisée pour l'émission. Le stimulateur cardiaque décrit dans ce document peut donc fonctionner en double monopolaire pour la première variante, et en bifocal pour la seconde variante.

Cependant, aucun de ces documents ne décrit un stimulateur destiné à fonctionner au choix selon les modes bipolaire, monopolaire double, double monopolaire ou bifocal ventriculaire.

Egalement, l'invention vise à résoudre le problème qui consiste à pouvoir utiliser d'autres modes de fonctionnement tels que double monopolaire ou bifocal ventriculaire.

L'invention consiste en un stimulateur cardiaque (1) implantable polyvalent par commande externe comprenant en combinaison un boîtier (3), conducteur de l'électricité, susceptible de constituer une borne d'un tripole d'excitation (T) qui comporte trois bornes (Tb, Tea, Teb); une source d'énergie électrique (4) logée dans le boîtier; des circuits et composants électroniques (5), également logés dans le boîtier, associés à la source d'énergie électrique, propres à délivrer des impulsions de stimulation du cœur du patient recevant ce stimulateur, comportant un amplificateur d'entrée (11) et un amplificateur de sortie (12), des moyens de commutation (15) commandés par des moyens de commande (16) externes, de façon à permettre par action sur ces moyens de commande (16) de passer d'un mode de fonctonnement possible du stimulateur à un autre mode de stimulation possible, deux sorties (6a, 6b) associées aux circuits et composants électroniques auxquelles peuvent être associées deux électrodes (8a, 8b) de stimulation comportant deux autres bornes du tripôle d'excitation, l'amplificateur d'entrée (11) et l'amplificateur de sortie (12) définissant un quadripôle de sortie (Q) à quatre bornes (Qsa, Qsb, Qea et Qed), les moyens de commutation (15) étant interposés entre le quadripôle de sortie Q et le tripôle d'excitation (T) (propre à permettre une pluralité de combinaisons de jonctions entre le quadripôle (Q) et le tripôle (T) caractérisé en ce que les moyens de commutation (15) comportent douze commutateurs (K1, K2, K3, K4, K5, K6, K7, K8, K9, K10, K11, K12) assurant, grâce à des moyens de connexion électriques (17) différentes combinaisons de liaison électrique possibles entre les quatre bornes (Qsa, Qsb, Qea et Qed) du quadripôle (Q) et les trois bornes (Tb, Tea et Teb) du tripôle (T), de façon à permettre, par action sur les moyens de commande (16), de sélectionner le fonctionnement du stimulateur parmi les modes monopolaire ou bipolaire, ou double monopolaire, ou monopolaire double, ou bifocal ventriculaire.

La présente invention présente comme avantage de permettre d'utiliser au mieux les différents modes de fonctionnement possibles d'un stimulateur du type décrit en fonction des besoins du patient et ce par voie externe c'est-à-dire de façon simple et sans recours à une opération chirurgicale. En outre, l'invention procure ces avantages sans accroissement notable de la complexité du stimulateur. Enfin, l'invention permet d'autres modes de fonctionnement possible du stimulateur.

L'invention sera bien comprise grâce à la description d'une forme d'exécution possible et non limitative en référence aux dessins annexés dans lesquels:

Les figures 1 à 5 sont cinq vues schématiques du stimulateur cardiaque suivant la présente invention dans cinq des modes de fonctionnement possibles;

la fig. 6 est une vue schématique des composants électroniques du stimulateur cardiaque suivant l'invention et de leurs connexions;

la fig. 7 est une vue schématique des commutateurs et des interconnexions constituant les moyens de commutation du stimulateur cardiaque suivant l'invention;

la fig. 8 est une représentation de l'état des moyens de commutation du stimulateur suivant l'invention dans un mode de fonctionnement possible.

L'invention concerne un stimulateur cardiaque 1 destiné à être implanté chez un patient dont la barrière matérielle que constitue la peau est représentée schématiquement par le trait continu 2.

Le stimulateur cardiaque 1 comporte un boîtier 3, conducteur de l'électricité, dans lequel sont logés d'une part une source d'énergie électrique 4 et d'autre part des circuits et composants électroniques 5, représentés seulement schématiquement. Le stimulateur cardiaque 1 comporte aussi deux sorties monopolaires 6a, 6b.

Le boîtier 3 et les deux sorties 6a, 6b sont reliés électriquement par des moyens de connexions électriques 7 représentés schématiquement aux trois bornes Tb, Tea et Teb d'un tripôle d'excitation T.

A chacune des deux sorties 6a, 6b est associée une électrode de stimulation monopolaire respectivement 8a, 8b, représentée schématiquement. Les extrémités libres 9a, 9b des électrodes 8a, 8b sont placées en deux points fixés et prédéterminés du cœur du patient, appartenant notamment à une même zone 10 de celui-ci, telle que le ventricule.

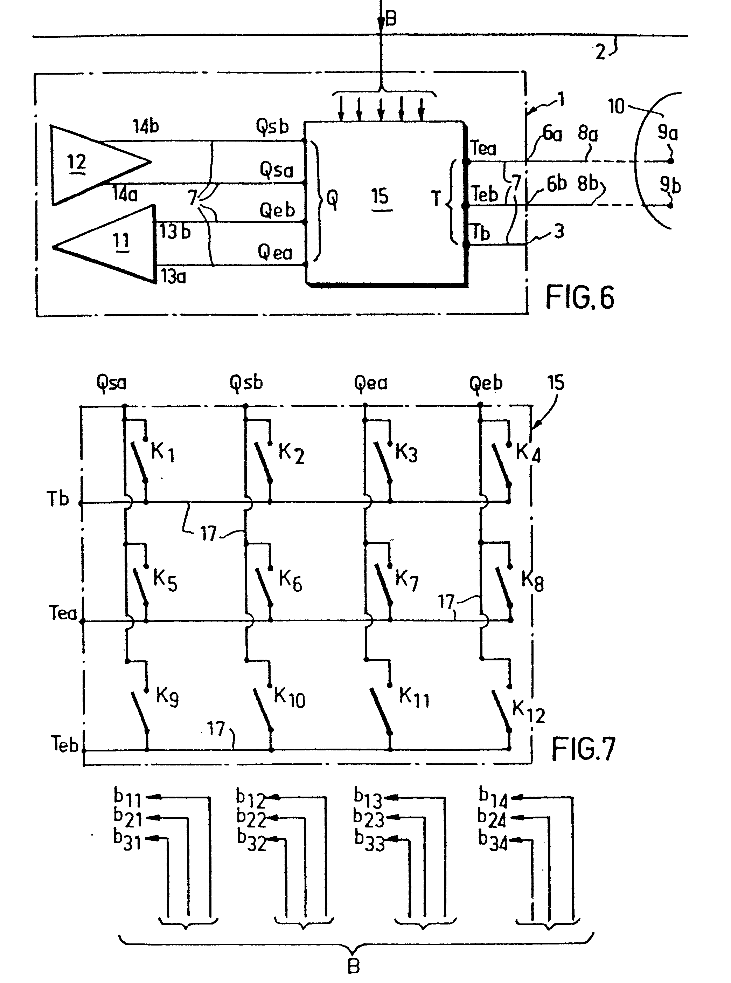

Les circuits et composants électroniques 5 sont associés à la source d'énergie électrique 4 et comportent notamment (figure 6) un amplificateur d'entrée 11 et un amplificateur de sortie 12 ayant chacun deux sorties respectivement 13a, 13b et 14a, 14b. Ces sorties sont reliées électriquement par des moyens de connexions électriques telles que 7 respectivement aux quatre bornes Qea, Qeb, Qsa et Qsb d'un quadripôle de sortie Q.

Le stimulateur cardiaque 1 comporte en outre des moyens de commutation 15 interposés entre le quadripôle de sortie Q et le tripôle d'excitation T en vue de permettre une pluralité de combinaison de jonctions entre les bornes Qea, Qeb, Qsa, Qsb du quadripôle et les bornes Tb, Tea, Teb du tripôle.

Les combinaisons de jonction dont il s'agit concernent les trois bornes du tripôle T ou seulement deux bornes de ce tripôle choisies parmi les trois, comme cela sera expliqué par la suite.

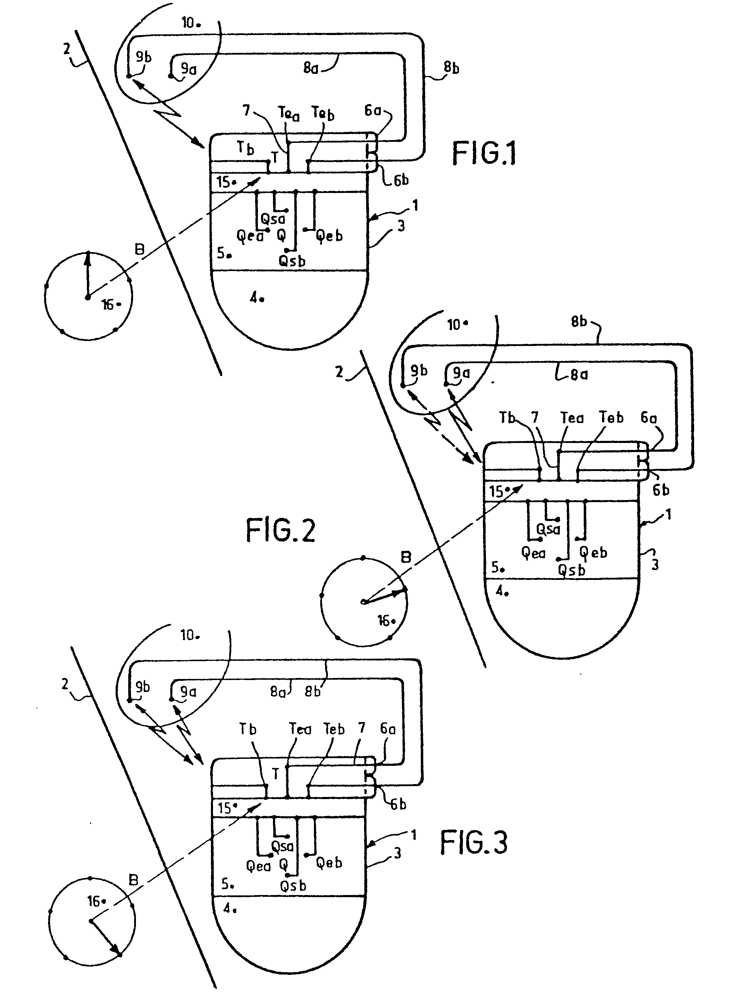

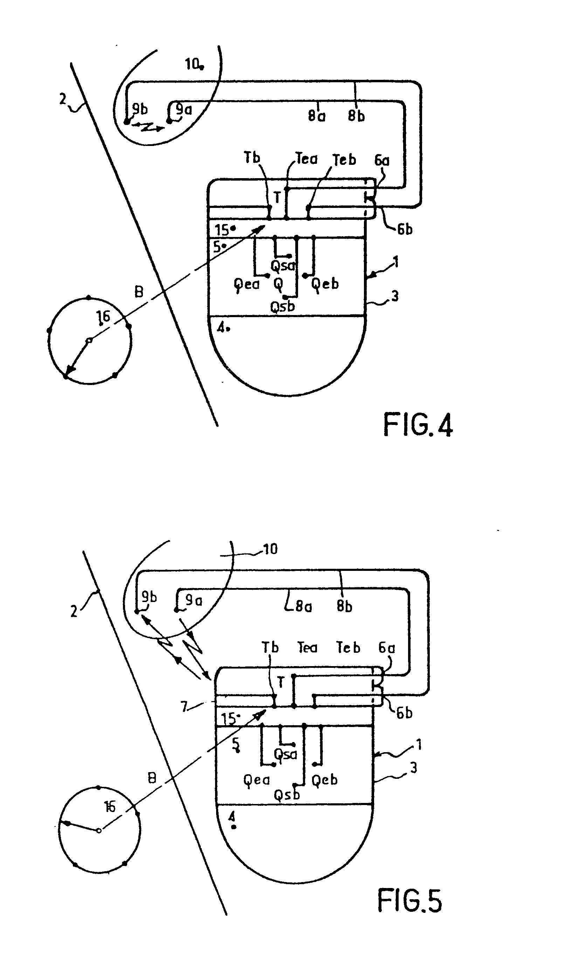

Les combinaisons de jonction entre le quadripôle Q et le tripôle T correspondent notamment à différents modes de fonctionnement du stimulateur à savoir le mode monopolaire (figure 1), double monopolaire (figure 2), monopulaire double (figure 3), bipolaire (figure 4), et «bifocal ventriculaire» (figure 5).

Suivant l'invention, les moyens de commutation 15 sont commandés depuis l'extérieur (c'est-à-dire du côté extérieur de la peau 2) par des moyens de commandes externes 16, de façon à permettre par simple action externe sur les moyens de commande 16 de passer d'un mode de fonctionnement possible du stimulateur cardiaque 1 à un autre mode de fonctionnement possible sans effraction de la peau 2. A cet effet, les moyens de commande 16 sont susceptibles de produire un train de bits de commande B permettant de placer les moyens de commutation 15 dans la position correspondant au mode de fonctionnement souhaité pour le stimulateur cardiaque 1.

Le mode de fonctionnement monopolaire est illustré par la figure 1. Dans ce mode, une seule des deux électrodes – soit par exemple l'électrode 8b – est active tandis que l'autre électrode – en l'occurrence 8a – est passive. Bien que passive, cette électrode est néanmoins mise en place, comme déjà mentionné, afin de permettre son utilisation dans les autres modes de fonctionnement possibles. Dans le mode de fonctionnement monopolaire, l'impulsion de stimulation du cœur intervient entre l'électrode active – soit l'extrémité 9b de l'électrode 8b – et le boîtier 3.

Le mode de fonctionnement double monopolaire est illustré par la figure 2. Dans ce mode, on met à profit la présence de l'électrode précédemment passive – en l'occurence l'électrode 8a – pour basculer le fonctionnement du stimulateur sur cette électrode en tant que de besoin notamment dans l'hypothèse non limitative d'une rupture de l'électrode 8b.

En pratique donc, le stimulateur est implanté avec les deux électrodes monopolaires 8a, 8b et peut fonctionner selon le mode monopolaire d'abord sur l'une des deux électrodes puis sur l'autre. Les deux électrodes 8a, 8b sont donc actives successivement.

Le mode de fonctionnement monopolaire double est illustré par la figure 3. Dans ce mode, les deux électrodes 8a, 8b sont actives simultanément, soit de façon équivalente, soit de façon différenciée. Les impulsions de stimulation interviennent donc entre le boîtier 3 et les extrémités 9a, 9b des deux électrodes 8a, 8b. Le boîtier est donc également actif.

Le mode bipolaire est illustré par la figure 4. Dans ce mode, le boîtier 3 est passif et les électrodes 8a, 8b actives, les impulsions de stimulation intervenant entre leurs extrémités 9a, 9b.

Quant au mode appelé «bifocal ventriculaire», il est illustré par la figure 5. Ce mode correspond au mode monopolaire double déjà décrit dans lequel chaque électrode 8a; 8b est unidirectionnelle au lieu d'être bidirectionnelle comme c'est le cas dans chacun des modes monopolaire, double monopolaire, monopolaire double et bipolaire décrits précédemment. Ainsi, dans le mode bifocal ventriculaire, l'une des deux électrodes, soit par exemple l'électrode 8a est exclusivement destinée à la réception tandis que l'autre électrode 8b est exclusivement destinée à l'excitation cardiaque. On conçoit naturellement que le rôle des deux électrodes 8a, 8b puisse être permuté. On conçoit également que ce cas est applicable à d'autres zones du cœur que le ventricule.

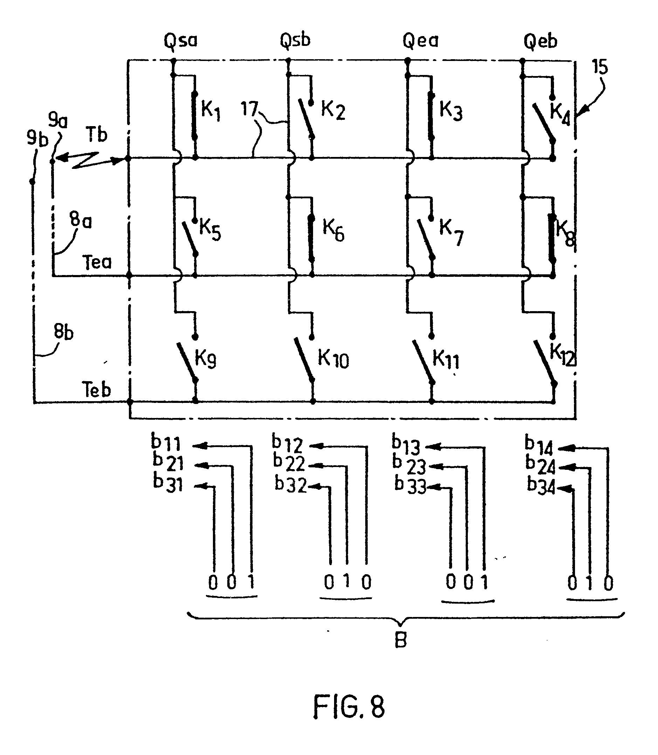

Les moyens de commutation 15 comprennent une batterie de douze commutateurs K1, K2, K3, K4, K5, K6, K7, K8, K9, K10, K11 et K12 (figure 7).

Cette batterie de commutateurs K1 à K12 et des moyens de connexions électriques 17 permettent d'assurer les différentes combinaisons de connexions électriques entre chaque borne Tb, Tea, Teb du tripôle T et chaque borne Qea, Qeb, Qsa et Qsb du quadripôle Q. Par exemple et comme illustré par la figure 7, le commutateur K1 est placé entre les bornes Tb et Qsa. Le commutateur K2 assure une liaison entre les bornes Tb et Qsb. Les autres commutateurs assurent les liaisons suivantes: K3 entre Tb et Qea, K4 entre Tb et Qeb, K5 entre Tea et Qsa, K6 entre Tea et Qsb, K7 entre Tea et Qea, K8 entre Tea et Qeb, K9 entre Teb et Qsa, K10 entre Teb et Qsb, K11 entre Teb et Qea enfin K12 entre Teb et Qeb (figure 7).

Les moyens de commande 16 peuvent faire l'objet de nombreuses formes d'exécution distinctes. En ce qui concerne le train de bits B, de nombreuses variantes peuvent être envisagées. Une forme d'exécution possible est celle illustrée par la figure 7 dans laquelle le train de bits B comporte une quadruple série de trois bits successifs à savoir b11, b21, b31 pour la première série, b1 2, b2 2, b3 2 pour la deuxième série, b1 3, b2 3, b3 3 pour la troisième série, b1 4, b2 4, b3 4 pour la quatrième et dernière série. Les bits de la première série commandent par exemple les commutateurs associés à la borne Qsa soit respectivement K1, K5, K9. Les bits de la deuxième série commandent les commutateurs associés à la borne Qsb, à savoir K2, K6 et K10. Les bits de la troisième série commandent les commutateurs associés à la borne Qea, à savoir K3, K7 et K11. Quant aux bits de la quatrième et dernière série ils commandent les commutateurs associés à la borne Qeb à savoir K4, K8 et K12.

Naturellement, on peut concevoir tout autre agencement notamment une triple série de quatre bits, chacune des séries commandant les commutateurs associés aux bornes Tb, Tea et Teb.

Toute autre combinaison en ce qui concerne la commande des commutateurs K1 à K12 peut aussi être envisagée.

Ainsi, les commutateurs K1, ... K12 peuvent être commutés de la manière suivante:

Pour le fonctionnement

1) monopolaire sur l'électrode 8a K1, K3, K6, K8 fermés, les autres étant ouverts.
2) monopolaire sur l'électrode 8a inverse K2, K4, K5, K6 fermés.
3) monopolaire sur l'électrode 8b K1, K3, K10, K12 fermés.
4) monopolaire sur l'électrode 8b inverse K2, K4, K9, K11 fermés.
5) double monopolaire K1, K3, K6, K8, K10 et K12 fermés.
6) bipolaire K5, K7, K10 et K12 fermés.
7) bipolaire K6, K8, K9 et K11 fermés.
8) bifocal ventriculaire K1, K3, K8, K10 fermés.

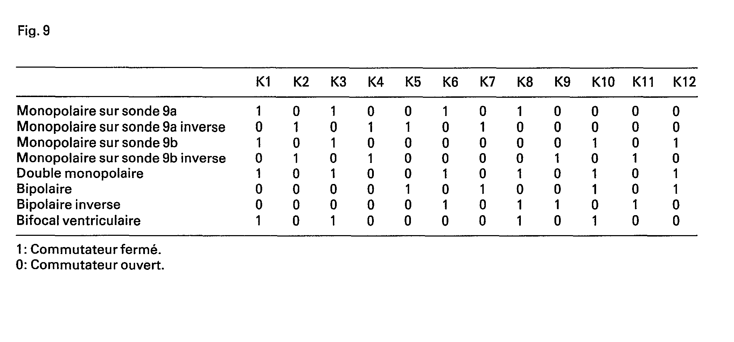

La figure 9 montre dans quels cas les commutateurs sont ouverts ou fermés.

Sur la figure 8, on a représenté schématiquement le mode de fonctionnement monopolaire sur l'électrode 8a. On a également indiqué de façon schématique les valeurs des bits de commande, le chiffre 0 indiquant que le commutateur reste ouvert et n'assure donc aucune liaison électrique alors que le chiffre 1 indique au contraire que le commutateur est fermé et permet la connexion. Le train de bits B est alors le suivant: 1, 0, 0 pour la première série, 0, 1, 0 pour la deuxième série, 0, 1, 0 pour la troisième série; 1, 0, 0 pour la quatrième et dernière série.

On comprend que pour faire basculer le stimulateur en mode de fonctionnement monopolaire sur l'autre électrode 8b, il convient de manœuvre les moyens de commande 16 pour appliquer aux moyens de commutation 15 le train de bits suivant: 1 0 0; 0 0 1, 0 0 1; 1 0 0.

A titre d'exemples, on peut encore mentionner que le train de bits permettant d'atteindre le fonctionnement en mode bipolaire est le suivant: 0 1 0; 0 0 1, 0 0 1, 0 1 0.

En ce qui concerne le mode monopolaire double, le train de bits est 1 0 0, 0 1 1, 0 1 1, 1 0 0. Pour le mode bifocal ventriculaire, le train de bits est 1 0 0; 0 0 1; 0 1 0; 1 0 0.

Naturellement, l'invention peut faire l'objet de nombreuses autres formes d'exécution. Egalement, il est clair que l'invention peut être étendue au cas de stimulateur comportant deux sorties chacune bipolaire.

## Revendications

1. Stimulateur cardiaque (1) implantable polyvalent par commande externe comprenant en combinaison un boîtier (3), conducteur de l'électricité, susceptible de constituer une borne d'un tripole d'excitation (T) qui comporte trois bornes (Tb, Tea, Teb); une source d'énergie électrique (4) logée dans le boîtier, des circuits et composants électroniques (5), également logés dans le boîtier, associés à la source d'énergie électrique, propres à délivrer des impulsions de stimulation du cœur du patient recevant ce stimulateur, comportant un amplificateur d'entrée (11) et un amplificateur de sortie (12), des moyens de commutation (15) commandés par des moyens de commande (16) externes, de façon à permettre par action sur ces moyens de commande (16) de passer d'un mode de fonctionnement possible du stimulateur à un autre mode de stimulation possible, deux sorties (6a, 6b) associées aux circuits et composants électroniques auxquelles peuvent être associées deux électrodes (8a, 8b) de stimulation comportant deux autres bornes du tripôle d'excitation, l'amplificateur d'entrée (11) et l'amplificateur de sortie (12) définissant un quadripôle de sortie (Q) à quatre bornes (Qsa, Qsb, Qea et Qed), les moyens de commutation (15) étant interposés entre le quadripôle de sortie Q et le tripôle d'excitation (T) (propre à permettre une pluralité de combinaisons de jonctions entre le quadripôle (Q) et le tripôle (T), caractérisé en ce que les moyens de commutation (15) comportent douze commutateurs (K1, K2, K3, K4, K5, K6, K7, K8, K9, K10, K11, K12) assurant, grâce à des moyens de connexion électriques (17) différentes combinaisons de liaison électrique possibles entre les quatre bornes (Qsa, Qsb, Qea et Qed) du quadripôle (Q) et les trois bornes (Tb, Tea et Teb) du tripôle (T), de façon à permettre, par action sur les moyens de commande (16), de sélectionner le fonctionnement du stimulateur parmi les modes monopolaire ou bipolaire, ou double monopolaire, ou monopolaire double, ou bifocal ventriculaire.

2. Stimulateur cardiaque suivant la revendication 1, caractérisé par le fait que les moyens de commande externes (16) sont susceptibles d'engendrer un train de bits de commande (B) des commutateurs constituant les moyens de commutation (15).

## Patentansprüche

1. Implantierbarer polyvalenter Herzschrittmacher (1) mit Aussenkommando, der gemeinsam ein elektrisch leitendes Gehäuse (3), das eine Klemme für einen Erregertripol (T), welcher drei Anschlüsse ($T_b$, $T_{ea}$, $T_{eb}$) aufweist, zu bilden imstande ist, eine in dem Gehäuse untergebrachte elektrische Energiequelle (4), ebenfalls in dem Gehäuse untergebrachte elektronische Schaltungen und Bauteile (5), die mit der elektrischen Energiequelle verbunden sind und zur Abgabe von Stimulationsimpulsen an das Herz des diesen Schrittmacher aufnehmenden Patienten geeignet sind, aufweist, mit einem Eingangsverstärker (11) und einem Ausgangsverstärker (12), Schalteinrichtungen (15), welche von externen Kommandoeinrichtungen (16) gesteuert werden, so dass sie durch Einwirkung auf diese Kommandoeinrichtungen (16) gestatten, von einer möglichen Betriebsart des Schrittmachers auf eine andere Stimulationsbetriebsart überzugehen und mit zwei Ausgängen (6a, 6b), welche mit den elektronischen Schaltungen und Bauteilen (5) verbunden sind und an welche zwei Stimulationselektroden (8a, 8b) angeschlossen werden können, welche zwei weitere Erregertripol-Klemmen aufweisen, wobei der Eingangsverstärker (11) und der Ausgangsverstärker (12) einen Ausgangsvierpol (Q) mit vier Klemmen ($Q_{sa}$, $Q_{sb}$, $Q_{ea}$ und $Q_{ed}$) bestimmen und die Schalteinrichtungen (15) so zwischen den Ausgangsvierpol (Q) und den Erregertripol (T) geschaltet sind, dass sie eine Vielzahl von Verbindungskombinationen zwischen dem Vierpol (Q) und dem Tripol (T) ermöglichen, dadurch gekennzeichnet, dass die Schalteinrichtungen (15) zwölf Schalter (K1, K2, K3, K4, K5, K6, K7, K8, K9, K10, K11, K12) aufweisen, die dank elektrischer Verbindungen (17) verschiedene Verbindungskombinationen zwischen den vier Klemmen ($Q_{sa}$, $Q_{sb}$, $Q_{ea}$ und $Q_{ed}$) des Vierpols (Q) und den drei Klemmen ($T_b$, $T_{ea}$ und $T_{eb}$) des Tripols (T) gewährleisten, so dass sie durch Einwirkung auf die Kommandoeinrichtungen (16) ermöglichen, die Betriebsart des Schrittmachers unter den Betriebsarten monopolar oder bipolar, doppelt monopolar, monopolar doppelt oder bifokal ventrikulär auszuwählen.

2. Herzschrittmacher nach Anspruch 1, dadurch gekennzeichnet, dass die externen Kommandoeinrichtungen (16) imstande sind, eine Folge von Kommandobits (B) an die Schalter abzugeben, die die Schalteinrichtungen (15) bilden.

**Claims**

1. A polyvalent implantable cadiac pacemaker (1) with external control means, comprising in combination an electricity conducting case (3) adapted to constitute one terminal of an excitation tripole (T) which comprises three terminals $(T_b, T_{ea}, T_{eb})$; an electrical power source (4) disposed in the case; electronic circuits and components (5) likewise disposed in the case, associated with the electrical power supply, adapted to deliver stimulation pulses to the heart of the patient receiving the pacemaker, comprising an input amplifier (11) and an output amplifier (12), switching means (15) controlled by external control means (16) for passing from one possible mode of operation of the pacemaker to another possible mode of stimulation, two outputs (6a, 6b) associated with the electronic circuits and components with which two stimulation electrodes (8a, 8b) can be associated comprising two other terminals of the excitation tripole, the input amplifier (11) and the output amplifier (12) defining an output quadripole (Q) with four terminals $(Q_{sa}, Q_{sb}, Q_{ea}$ and $Q_{ed})$, the switching means (15) being interposed between the output quadripole (Q) and the excitation tripole (T), adapted to allow a plurality of junction combinations between the quadripole (Q) and the tripole (T) characterised in that the switching means (15) comprise twelve switches (K1, K2, K3, K4, K5, K6, K7, K8, K9, K10, K11, K12) providing, with the aid of the electrical connection means (17), various combinations of possible electrical connections between the four terminals $(Q_{sa}, Q_{sb}, Q_{ea}$ and $Q_{ed})$ of the tripole (Q) and the three terminals $(T_b, T_{ea}$ and $T_{eb})$ of the tripole (T), so as to allow, by acting on the control means (16), to select the operation of the pacemaker from the monopolar or bipolar modes, or monopolar double, or double monopolar, or ventricular bifocal.

2. A cardiac pacemaker according to claim 1, characterised in that the external control means (16) are adapted to generate a train of control bits (B) for the switches comprising the switching means (15).

FIG.1

FIG.2

FIG.3

FIG.4

FIG.5

FIG.6

FIG.7

FIG. 8

Fig. 9

|  | K1 | K2 | K3 | K4 | K5 | K6 | K7 | K8 | K9 | K10 | K11 | K12 |
|---|---|---|---|---|---|---|---|---|---|---|---|---|
| Monopolaire sur sonde 9a | 1 | 0 | 1 | 0 | 0 | 1 | 0 | 1 | 0 | 0 | 0 | 0 |
| Monopolaire sur sonde 9a inverse | 0 | 1 | 0 | 1 | 1 | 0 | 1 | 0 | 0 | 0 | 0 | 0 |
| Monopolaire sur sonde 9b | 1 | 0 | 1 | 0 | 0 | 0 | 0 | 0 | 0 | 1 | 0 | 1 |
| Monopolaire sur sonde 9b inverse | 0 | 1 | 0 | 1 | 0 | 0 | 0 | 0 | 1 | 0 | 1 | 0 |
| Double monopolaire | 1 | 0 | 1 | 0 | 0 | 1 | 0 | 1 | 0 | 1 | 0 | 1 |
| Bipolaire | 0 | 0 | 0 | 0 | 1 | 0 | 1 | 0 | 0 | 1 | 0 | 1 |
| Bipolaire inverse | 0 | 0 | 0 | 0 | 0 | 1 | 0 | 1 | 1 | 0 | 1 | 0 |
| Bifocal ventriculaire | 1 | 0 | 1 | 0 | 0 | 0 | 0 | 1 | 0 | 1 | 0 | 0 |

1: Commutateur fermé.
0: Commutateur ouvert.